# EUROPEAN PATENT SPECIFICATION

(11) **EP 3 858 850 B1**
(45) Date of publication and mention of the grant of the patent: **28.02.2024**
(21) Application number: 19866300.7
(22) Date of filing: 13.09.2019
(51) Int. Cl.: C07K 7/06, A61K 38/08, A61P 7/10

(54) **NONAPEPTIDE PREVENTING INCREASED HYPERPERMEABILITY OF VASCULAR ENDOTHELIUM**
NONAPEPTID ZUR VORBEUGUNG EINER ERHÖHTEN HYPERPERMEABILITÄT DES GEFÄSSENDOTHELS
NONAPEPTIDE PERMETTANT DE PRÉVENIR LA HAUSSE D'HYPERPERMÉABILITÉ D'ENDOTHÉLIUM VASCULAIRE

(30) Priority: 26.09.2018 RU 2018133901
(43) Date of publication of application: 04.08.2021
(73) Proprietor: Federalnoye Gosudarstvennoe Budgetnoe Uchrezhdenie "Nacionalniy Medicinskiy Issledovatelskiy Centr Kardiologii imeni akademika E.I. Chazova", Moscow, 121552 (RU)
(72) Inventor: ARZAMASCEV, Yevgeniy Veniaminovich, Moscow, 119526 (RU); AZMUKO, Andrey Andreevich, Moscow, 121359 (RU); BUSHUEV, Valeri Nikolaevich, Moscow, 121351 (RU); KAZAKOVA, Olga Alekseevna, Moscow, 119121 (RU); MOLOKOEDOV, Aleksandr Sergeevich, Moscow, 125424 (RU); OVCHINNIKOV, Mihail Vladimirovich, Moscow, 115162 (RU); PALKEEVA, Marina Yevgenievna, Moscow, 121614 (RU); SIDOROVA, Mariya Vladimirovna, Moscow, 125424 (RU); SAMSONOV, Mikhail Vasilievich, Lyubertsy, 140000 (RU); TEREKHOVA, Olga Alexandrovna, Moscow, 121170 (RU); KHAPCHAEV, Asker Yusufovich, Moscow, 125047 (RU); SHIRINSKY, Vladimir Pavlovich, Moscow, 125252 (RU)
(74) Representative: Meissner Bolte Partnerschaft mbB
(86) International application number: PCT/RU2019/000635
(87) International publication number: WO 2020/067923

(56) References cited:
- RU-C1- 2 402 565
- RU-C1- 2 493 164
- ADESSI C ET AL: "CONVERTING A PEPTIDE INTO A DRUG: STRATEGIES TO IMPROVE STABILITY AND BIOAVAILABILITY", CURRENT MEDICINAL CHEMISTRY, BENTHAM, NL, vol. 9, 1 May 2002 (2002-05-01), pages 963-978, XP009061547, ISSN: 0929-8673, DOI: 10.2174/0929867024606731
- KHAPCHAEV A Y ET AL: "Suppression of vascular endothelium hyperpermeability by cell-permeating peptide inhibitors of myosin light chain kinase", BIOPHYSICS, SP MAIK NAUKA/INTERPERIODICA, DORDRECHT, vol. 57, no. 5, 29 December 2012 (2012-12-29), pages 587-591, XP035154867, ISSN: 1555-6654, DOI: 10.1134/S0006350912050089
- SIÂN-ELERI OWENS ET AL: "A Strategy to Identify Stable Membrane-Permeant Peptide Inhibitors of Myosin Light Chain Kinase", PHARMACEUTICAL RESEARCH, KLUWER ACADEMIC PUBLISHERS-PLENUM PUBLISHERS, NL, vol. 22, no. 5, 1 May 2005 (2005-05-01), pages 703-709, XP019370840, ISSN: 1573-904X, DOI: 10.1007/S11095-005-2584-9
- John T Hunt , David M Floyd , Vinh G Lee , Deborah K Little , Suzanne Moreland: "Minimum requirements for inhibition of smooth-muscle myosin light-chain kinase by synthetic peptides", Biochemical Journal, vol. 257, no. 1, 1 January 1989 (1989-01-01), pages 73-78, XP055700122, DOI: 10.1042/bj2570073
- Xu Guozhong, Takamoto Keiji, Chance Mark R.: "Radiolytic Modification of Basic Amino Acid Residues in Peptides: Probes for Examining Protein-Protein Interactions", Analytical Chemistry, vol. 75, no. 24, 7 November 2003 (2003-11-07), pages 6995-7007, XP055805082, ISSN: 0003-2700, DOI: 10.1021/ac035104h
- Richard B Pearson , Mufti Y Misconi , Bruce E kemp: "Smooth Muscle Myosin Kinase Requires Residues on the COOH-terminal Side of the Phosphorylation Site. Peptide inhibitors", The journal of biological chemistry, vol. 261, no. 1, 5 January 1986 (1986-01-05), pages 25-27, XP055700139,
- James Bonner , Yana A Lyon , Christoffer Nellessen , Ryan R Julian: "Photoelectron Transfer Dissociation Reveals Surprising Favorability of Zwitterionic States in Large Gaseous Peptides and Proteins", Journal of the American Chemical Society, vol. 139, no. 30, 2 August 2017 (2017-08-02), pages 10286-10293, XP055700142, DOI: 10.1021/jacs.7b02428
- J. D. Strauss, P. de Lanerolle, R. J. Paul: "Effects of myosin kinase inhibiting peptide on contractility and LC20 phosphorylation in skinned smooth muscle", American journal of physology, vol. 262, no. 6, 1 June 1992 (1992-06-01), pages C1437-C1438, XP009527560, ISSN: 0363-6143, DOI: 10.1152/ajpcell.1992.262.6.C1437

## Description

The invention relates to a biologically active peptide, which is able to prevent stress-induced vascular hyperpermeability. This property may render the peptide to be used in therapeutic interventions as an anti-edematous agent.

Acute changes in the vascular permeability are mediated by vascular endothelial lining. Endothelium is a barrier between blood and the underlying tissues to selectively deliver nutrients, transmit humoral signals, and excrete metabolic products. Various stress agents like bacterial endotoxins, ischemia, necrotoxins, reactive oxygen species, nitrosyl compounds, mechanical trauma, etc. disrupt contacts between endothelial cells and induce endothelial cell contraction. When the endothelial barrier function is compromised, proteins and cells permeate tissues from blood yielding acute edema and provoking local activation of immune cells. Lung or brain edema may lead to life-threatening conditions. Ischemia and subsequent reperfusion of myocardium and other organs also induce local vascular permeability accompanied by postischemic edema and neutrophil infiltration, which damage tissues. In addition, in some coronary vessels, the blood-flow is not restored after ischemia/reperfusion (the 'no reflow' phenomenon). This condition is also attributed to endothelial dysfunction, in particular with capillary endothelium contraction and reduction in capillary diameter.

Myosin light chain kinase (MLCK) is a validated enzyme to induce endothelial hyperpermeability. Endothelial cells express the 210 kDa MLCK isoform. Various stress agents activate endothelial MLCK and initiate intercellular contact disassembly and endothelial cell retraction. Mice with genetic MI,CK-210 knockout are less susceptible to lung injury in experimental sepsis models because their microvascular endothelial barrier function is preserved. In sepsis models, MLCK inhibitors protect wild-type mice lungs similar to effects of the genetic MLCK knockout. These data suggest pharmacological inactivation of microvascular endothelial MLCK may slow down tissue edema development and tissue injury.

Most of MLCK inhibitors based on low molecular weight organic compounds that are used in experimental studies are not suitable for medical use because of multiple side effects. Therefore, there is a demand for development of pharmacologically privileged molecules suitable for human use as anti-edematous agents. In particular, peptides generally belong to such compounds.

Pearson et al. (1986) synthesized **H-Lys-LysArg-Ala-Ala-Arg-Ala-Thr-Ser-NH₂ (MLC**_{**11**-}**₁₉)** peptide, which corresponds to myosin regulatory light chain (MLC) amino acids 11-19 and proves superior as a smooth muscle MLCK inhibitor as compared to other MLC-based peptides in an experimental setting with purified proteins in vitro. MLC₁₁₋₁₉ decreases MLC phosphorylation in neutrophils indicating it is cell-permeant (Chilcoat et al., 2008). The peptide MLC₁₁₋₁₉ is a structural analogue of the claimed compound. Due to its chemical structure, MLC₁₁₋₁₉ is extremely prone to proteolytic degradation; therefore, it is unlikely MLC₁₁₋₁₉ would ever be used in medical practice.

The PIK (**P**eptide **I**nhibitor of **K**inase) family peptides are also known that are based on the MLCK autoinhibitory sequence. Nonapeptide **H-Arg-Lys-Lys-Tyr-Lys-Tyr-Arg-Arg-Lys-NH₂ (L-PIK)** (Lukas et al., 1999) permeates plasmalemma and affects epithelial permeability both in vitro and in vivo (Clayburgh et al., 2005). L-PIK, like MLC₁₁₋₁₉, is highly susceptible to proteolytic degradation (Owens et al., 2005) rendering it unlikely to be used in medical practice. As expected, L-PIK modification via substitution of L- amino acids by their D-analogues increased resistance of the peptide to proteolysis (Clayburgh et al., 2005). However, the inhibitory activity of D-PIK was significantly lower as compared to that of L-PIK, apparently because D-PIK, as a complete L-PIK enantiomer, has different spatial structure (Sekridova et al., 2010). Comparison of various MLCK peptide inhibitors that were obtained as modifications of the original L-PIK, namely D-PIK, [N^{α}Me-Arg¹]-PIK, [εAca¹]-PIK, [Citl]-PIK, [Cit¹,Orn³]PIK), demonstrated that only **H-MeArg¹-Lys-Lys-Tyr-Lys-Tyr-Arg-Arg-Lys-NH₂ [N^{α}MeArg¹]-PIK** (designated PIK1), which carried a modification of the L-PIK N-terminal amino acid, had an in-vitro activity comparable to that of L-PIK (Russian Federation patent 2402565, CO7K 7/00, issued 27.10.2010). PIK1 half-life in human plasma in vitro is almost 3-fold higher as compared to that of L-PIK. Thrombin-mediated endothelial hyperpermeability is inhibited 1.6-fold when PIK1 is present at a concentration of 100 µM.

Relatively high working concentrations rendered PIK1 less attractive for use in vivo. Next, PIK1 composition was modified further with D-Arg⁸ instead of L-Arg⁸. The modified MLCK inhibitor was designated PIK2 (Russian Federation patent 2493164, C07K 7/06, issued 20.09.2013). While PIK2 stability was 4-fold higher as compared to PIK1, the introduced modification did not affect the inhibitory properties of PIK2. That is, PIK2 completely abolished the thrombin-mediated endothelial hyperpermeability in vitro at concentrations of 20 µM, i.e. 5-fold less as compared to PIK1. Collectively, PIK2 was superior to PIK1 in both stability and biological activity. PIK2 is considered as a prototype of the claimed compound. However, experimental animals demonstrated low tolerance after single intravenous injection of PIK2. That is, in mice, PIK2 LD₅₀ was 18 mg/kg whereas in Chinchilla rabbits, LD₅₀ was not more than 5 mg/kg indicating a narrow therapeutic window for PIK2. Thus, despite achieving the aimed effect, i.e. abrogation of the endothelial hyperpermeability response and lung edema in rats PIK2 exhibited low chances for medicinal use due to relatively high toxicity.

The aim of the invention is the development of proteolytically stable nonapeptide able to prevent the vascular endothelium hyperpermeability for use as a peptide preparation that combines high biostability with low toxicity to provide for a wide therapeutic window in estimated use in medical practice.

Relevance of the task is stipulated by the need to supply the emergency medicine with effective and safe anti-edematous and endothelial protective drugs that are obviously lacking in the relevant segment of the pharmaceutical market.

The technical result of the invention is the synthesis of the peptide with a formula (1), which effectively decreases vascular endothelial hyperpermeability and features by low toxicity and prolonged lifetime in human plasma.

The technical result is achieved by the synthesis of nonapeptide amide of formula

**H-Lys¹¹-D-Lys¹²-Arg¹³-Ala¹⁴-Ala¹⁵-Arg¹⁶-Ala¹⁷-Thr¹⁸-Ser¹⁹-NH₂** **(1),**

**or [D-Lys¹²]-MLC₁₁₋₁₉ (1)**, hereafter referred as **PIK7,** where natural L-Lys¹² is substituted for D-Lys¹².

The claimed peptide, PIK7, and the control peptides, MLC₁₁₋₁₉ (a structural analogue) and PIK2 (a functional analogue taken as a prototype) were synthesized using a solid-phase peptide synthesis method utilizing the Fmoc-technology as described below in Example 1.

Non-obviousness of invention is determined by the fact that any peptide design is a clearly theoretical approach because any peptide that derives as a modification represents a unique chemical compound with non-obvious biological activity, which has to be validated experimentally.

Thus, substitution of native L-conformation of Lys¹² Cα atom to D-conformation should have increased the peptide half-life in plasma because peptide bonds formed by D-amino acids are more stable due to inability of proteolytic enzymes to identify these kind of peptide bonds (Hruby and Qian, 1994). However, along with an increased half-life, modifications might have exerted a negative effect on the inhibitory properties of the claimed peptide because among peptide analogues, there is no clear and predictable dependance between the structure and the biological activity. Even minor modifications (like changing the native L-conformation of Lys¹² Cα atom to D-conformation) might dramatically alter the peptide inhibitory properties. This notion is supported by an experimental fact that in a hexapeptide corresponding to RLC sequence 11-16, the N-terminal L-Lys residue substitution with D-Lys resulted in a significant decrease in the inhibitory properties. That is, the IC₅₀ values for H-**L**-Lys-Lys-Arg-Ala-Ala-Arg-NH₂ and H-**D-**Lys-Lys -Arg-Ala-Ala-Arg-NH₂ were 22 µM and 180 µM, respectively (Hunt et al., 1989).

The invention is illustrated by figures.

Fig. 1. Representative ¹H-NMR (500 MHz) spectra fragments illustrating the degradation patterns of MLC₁₁₋₁₉ (free plasma spectrum subtracted) in human plasma at 18 (b), 25 (c), 36 (d), 50 (e), and 65 (f) min; (a), the spectrum of MLC₁₁₋₁₉ in phosphate buffered saline, pH 7.4, D₂O.

Fig. 2. Representative ¹H-NMR (500 MHz) spectra fragments illustrating the degradation patterns of the claimed peptide (free plasma spectrum subtracted) in human plasma at 140 (b), 200 (c), and 330 (d) min; (a), the spectrum of PIK7 in phosphate buffered saline, pH 7.4, D₂O.

These spectra show relative resistance of both the analogue and the clamed peptide to plasma proteolytic enzymes as described in Example 2.

Fig. 3. The thrombin-mediated FITC-dextran permeability through the EA.hy926 endothelial cell monolayer in the presence of myosin light chain kinase peptide inhibitors. Thrombin (100 nM) was added at 0 min, the cells were preincubated for 60 min with MLCK peptide inhibitors (100 µM). Data are presented as mean±SD, n=5. p<0.05 for all data points within the 20-180 min time course range as compared to the thrombin-stimulated control.

Fig. 4. Inhibition of the thrombin-induced RLC phosphorylation in EA.hy926 endothelial cells by PIK2, PIK7 ([D-Lys¹²]-MLC₁₁₋₁₉), or MLC₁₁₋₁₉ MLCK peptide inhibitors. Inhibitors were added up to 100 µM, n=3-5. p<0.05 for PIK2, PIK7 vs control (0, 20 min). p<0.05 for PIK2 vs PIK7 (0, 20 min).

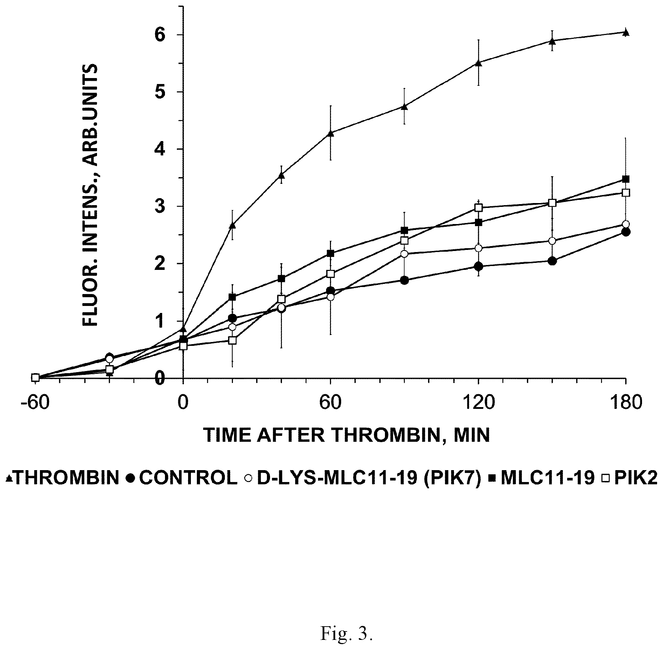

Effects of the claimed peptide (PIK7), the prototype (PIK2), and the analogue (MLC₁₁₋₁₉) on the endothelial permeability was assayed in a model of FITC-dextran diffusion through the endothelial monolayer as described in Example 3. Fig. 3 shows the dynamics of the thrombin-induced FITC-dextran permeability through the EA.hy926 endothelial cell monolayer in the presence of PIK7, PIK2, or MLC₁₁₋₁₉ myosin light chain kinase peptide inhibitors. Under these experimental conditions, all inhibitors tested in the assay completely abolished the FITC-dextran hyperpermeability whereas the inhibitory effect of PIK7 was not inferior to those of either the prototype, PIK2, or the analogue, MLC₁₁₋₁₉.

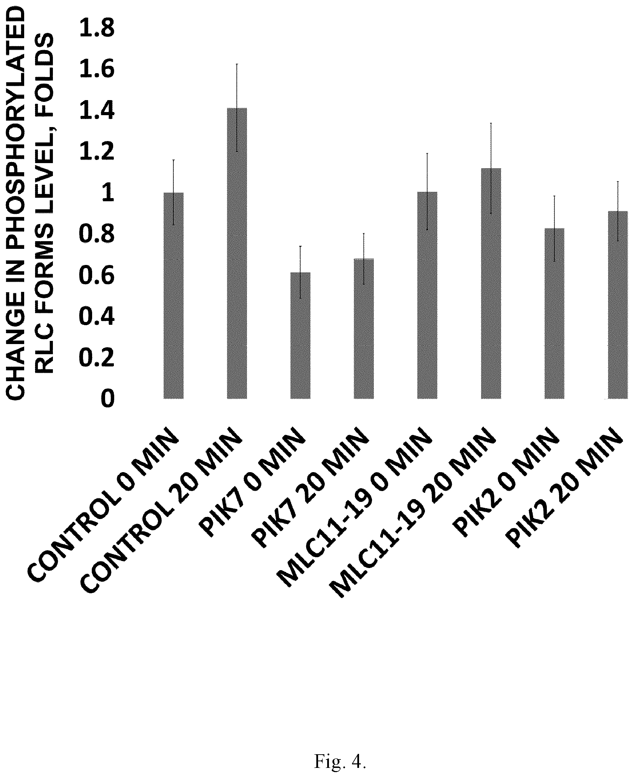

Inhibitory effects of the claimed peptide (PIK7), its prototype (PIK2), and analogue (MLC₁₁₋₁₉) were compared in an assay of myosin regulatory light chain (RLC) phosphorylation in vitro as described in Example 4. Fig. 4 shows the effects of the claimed peptide (PIK7), its prototype (PIK2), and analogue (MLC₁₁₋₁₉) on the thrombin-induced myosin regulatory light chain (RLC) phosphorylation in EA.hy926 endothelial cells. The claimed peptide, PIK7, inhibited myosin RLC phosphorylation in endothelial cells with efficacy higher than that of either the prototype, PIK2, or the analogue, MLC₁₁₋₁₉.

A comparison of the acute toxicity and tolerance of PIK7 and PIK2 was carried out in mice and rabbits as described in Example 5.

Assessment of the acute toxicity and tolerance of PIK2 and PIK7 in experimental animals undoubtedly indicates that the tolerance of PIK7 is more than one order of magnitude higher whereas the acute toxicity is lower. Therefore, PIK7 has a wider therapeutic window as compared to PIK2 (see Table 3).

Thus, comparative evaluation of the proteolytic stability, biological activity, and toxicity (tolerance) of the claimed peptide (PIK7) versus the peptides chosen as an analogue and a prototype shows that PIK7 is not inferior either to the structural analogue (MLC₁₁₋₁₉) or to the prototype (PIK2). That is, PIK7 is i) highly active as an inhibitor of vascular endothelial hyperpermeability, ii) superior to both PIK2 and MLC11-19 as an inhibitor of myosin RLC phosphorylation in endothelial cells, iii) more resistant to proteolysis (by one order of magnitude as compared to the structural analogue), and iv) significantly less toxic than the prototype (PIK2).

The invention is described by 5 (five) examples as follows.

### Example 1. Synthesis of the claimed peptide (PIK7)

**H-Lys-*D*-Lys-Arg-Ala-Ala-Arg-Ala-Thr-Ser-NH₂** **(I)**

Abbreviations:
AA, amino acid;
Boc, tert-butoxycarbonyl;
Bu^{t}, tert-butyl;
DCM, dichloromethane;
DMSO-*d₆*, dimethyl sulfoxide;
DMF, N,N-dimethylformamide;
Fmoc, 9-fluorenylmethoxycarbonyl;
HOBt, 1-hydroxybenzotriazol;
HPLC, reversed phase high performance liquid chromatography;
MALDI-TOF MS, matrix-assisted laser desorption and ionization time-of-flight mass spectrometry;
4-MePip, 4-methylpiperidin;
NMM, N-methylmorpholine;
¹H-NMR, proton nuclear magnetic resonance;
PIK, Peptide Inhibitor of Kinase;
Pbf, 2,2,4,6,7-pentamethyldihydrobenzofuran-5-sulfonyl;
TBTU, 2-(1H-benzotriazol-1-yl)-1,1,3,3-tetramethyluronium tetrafluoroborate;
TIBS, triisobutylsilane;
TFA, trifluoroacetic acid.

The derivative of Fmoc-amino acids and TBTU were obtained from NovaBiochem (Switzerland), NMM, HOBt, TIBS, 4-methylpiperidine, DMF, DCM, and TEA were from Fluka (Switzerland), acetonitrile was from Panreac (Spain). Analytical HPLC was performed on a Gilson chromatograph (France) on a Nucleosil 100-5 C18 column (Sigma, USA) (4.6 × 250 mm, 5 µm). The peptides were eluted in a gradient of buffer B in buffer A (from 0 to 60%) at a flow rate of 1 mL/min with UV detection at 220 nm. Buffer A: 0.1% TFA; buffer B: 80% acetonitrile in buffer A. Preparative HPLC was performed on a Knauer system (Germany) using Diasorb C16 130T columns (20 × 250mm, 10 µm). The elution was achieved with a linear gradient of aqueous 0.1% TFA (A) and 80% acetonitrile in water (B) at a flow rate of 10 mL/min with UV detection at 220 nm. Peptide structures were confirmed by both ¹H-NMR and mass-spectrometry techniques. ¹H-NMR spectra were recorded on a WH-500 Bruker 500 MHz spectrometer (Germany) in DMSO-*d₆* at 300 K, the concentration of peptides was 2-3 mg/mL. Chemical shifts were measured relative to tetramethylsilane. Mass-spectrometry data were obtained using an Ultrafex MALDI TOF/TOF instrument (Bruker Daltonics, Germany).

Peptide (I) was synthesized using a Tribute-UV peptide synthesizer (Protein Technologies Inc., USA) according to a standard protocol for single-coupling of Fmoc-amino acids using TBTU (see Table1). The synthesis of peptide amide was carried out on a Rink-amide resin (Novabiochem, Switzerland) with amino group density of 0.64 mmol/g. Amino acid side chain functional groups were blocked with acid-labile protecting groups designed for the Fmoc strategy: Boc at ε-amino groups of Lys; Bu^{t} at hydroxyl groups of Ser and Thr; Pbf at guanidine groups of Arg. The Fmoc-amino acids were attached step-by-step starting from the 0.38 mmol C-terminal residue. The final side chain deprotection and cleavage of peptide from a solid support was performed by treatment with a solution containing 95% TFA, 2.5% water, and 2.5% TIBS for 1.5 h at 20 °C. The polymer was filtered off, washed with the deprotection mixture (2 × 2 mL); the filtrate was evaporated followed by the addition of dry ether to the residue. The precipitate was filtered, washed with DCM (2 × 5 mL), ether (3 × 5 mL), and dried in a vacuum desiccator. The crude product (0.76 g, containing 52% of the target substance according to a HPLC assay) was purified by preparative HPLC. After lyophilization, 0.23 g of the peptide (I) was obtained with 97.2 % purity according to HPLC data. The yield was 38.8% per starting amino acid. Similarly, the structural analog (MLC₁₁₋₁₉) and the prototype (PIK2) were synthesized. The structures of the claimed peptide (PIK7), the prototype (PIK2), and the structural analogue (MLC₁₁₋₁₉) were confirmed by mass spectrometry and ¹H-NMR (see Table 2), and the purity was confirmed by analytical HPLC (see Tables 2, 3).

**Table 1. Protocol of solid phase peptide synthesis**

| No | Operation | Reagent | Treatment time, min |
|---|---|---|---|
| 1 | Deprotection of α-amino groups | 25% 4-MePip/DMF | 5 |
| 2. | Washing | 5 × DMF | 3 |
| 3. | Activation | 1.5 mmol Fmoc-AA + 1.5 mmol TBTU + 1.5 mmol NMM in DMF | 3 |
| 4. | Coupling | 1.5 mmol of activated derivative Fmoc-AA in DMF | 60 |
| 5. | Washing | 5 × DMF | 3 |

**Table 2. Chemical shifts (δ, ppm) of proton signals in the ¹H-NMR spectrum of PIK7 in DMSO-d₆ at 300 K**

| Amino acid residue | NH | α-CH | β-CH |
|---|---|---|---|
| Lys¹ | 8.15 | 3.79 | 1.70, 1.66 |
| Lys² | 8.54 | 4.29 | 1.66, 1.50 |
| Arg³ | 8.14 | 4.22 | 1.67, 1.51 |
| Ala⁴ | 8.01 | 4.27 | 1.20 |
| Ala⁵ | 8.00 | 4.27 | 1.20 |
| Arg⁶ | 7.90 | 4.37 | 1.67,1.50 |
| Ala⁷ | 8.10 | 4.36 | 1.23 |
| Thr⁸ | 7.83 | 4.28 | 4.04 |
| Ser⁹ | 7.74 | 4.19 | 3.65 |
| Other | 7.64, 7.18 CONH₂ | | |

**Table 3. Characteristics of the claimed peptide, prototype, and analogue**

| Peptide ID | Sequence | Molecular mass (calc.), g/mol | HPLC purity, % | MALDI-TOF MS, m/z | T_{1/2}, half-life in human plasma *in vitro* (min) | Toxicity LD₅₀ in mice/rabbits (mg/kg) |
|---|---|---|---|---|---|---|
| PIK2 (the prototype) | H-(*N*-Me)-Arg¹-Lys-Lys-Tyr-Lys-Tyr-Arg⁷-*D*-Arg⁸-Lys-NH₂ | 1338.65 | 96.8 | 1338.7 | >540 (Khapchaev et al., 2016) | 18/5 |
| PIK7 (the claimed peptide) | H-Lys-*D*-LysArg-Ala-Ala-Arg-Ala-Thr-Ser-NH₂ (I) | 987.17 | 97.2 | 987.09, 1009.06 [M+Na]⁺ | ≥320 | >1000/>10 |
| MLC₁₁₋₁₉ (the analogue) | H-Lys-LysArg-Ala-Ala-Arg-Ala-Thr-Ser-NH₂ | 987.17 | 96.1 | 987.20 | 32 | not determined |

### Example 2. Peptide half-lives in human plasma in vitro (based on ¹H-NMR spectroscopy data)

Whole blood (100 mL) was collected from healthy donors to 50-mL tubes containing 5 mL of a preservative (130 mM citrate in phosphate buffered saline (PBS), pH 7.4). The blood was incubated for 15-20 min at room temperature followed by centrifugation at 1500 g for 15 min at 18°C. The supernatants were transferred to clear tubes and centrifuged as above. Dry NaBr was added to the plasma (280 mg per 1 mL of plasma). After dissolution of NaBr, the plasma (approx. 30 mL) was transferred to centrifuge tubes (Type 45Ti rotor, Beckman, USA) and layered with 20 mL of a solution (specific mass of 1.216 g/L), which was prepared as follows - 11.42 g of NaCl and 100 mg of ethylenediaminetetraacetic acid (EDTA) were dissolved in distilled water in a final volume of 1 L (pH 7.4) and 281.44 g of NaBr was added. The plasma was centrifuged at 40,000 rpm for 48 hrs. The upper dark-yellow fraction containing lipoproteins and the colorless buffer fraction were discarded leaving approx. 1-cm high buffer above the apparent plasma border. Portions of plasma obtained from one donor were pooled and dialyzed for 36 hrs against 4 L of PSB free of Ca²⁺ and Mg²⁺ (MP Biomedicals, USA) in dialysis bags with 12-14 kDa pore sizes. The dialysis procedure was repeated twice with fresh buffer changed every 24 hrs. After the dialysis, the plasma was centrifuged at 25,000 rpm (Type 45Ti rotor, Beckman, USA) for 15 min at 4°C. The supernatant fraction was collected and lyophilized. The dry matter was dissolved in D₂O in a final volume equal to the plasma volume before NaBr was added, lyophilized again, dissolved in the same volume of D₂O, and stored in 1-mL aliquots at -20°C before use.

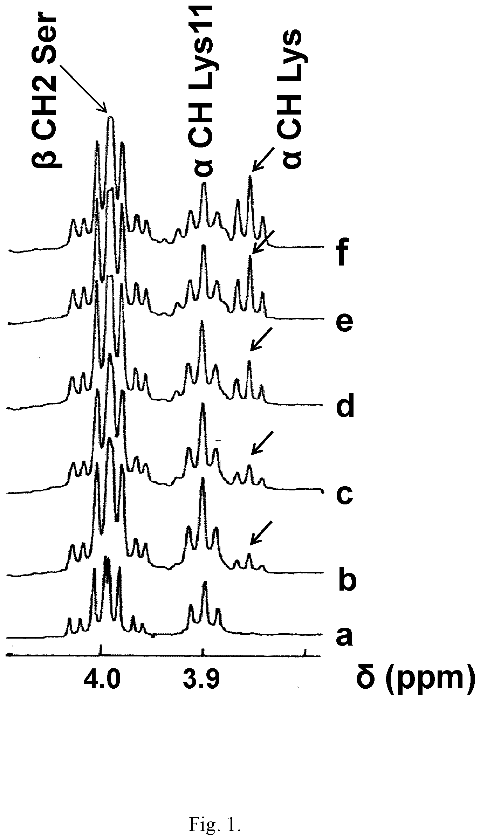

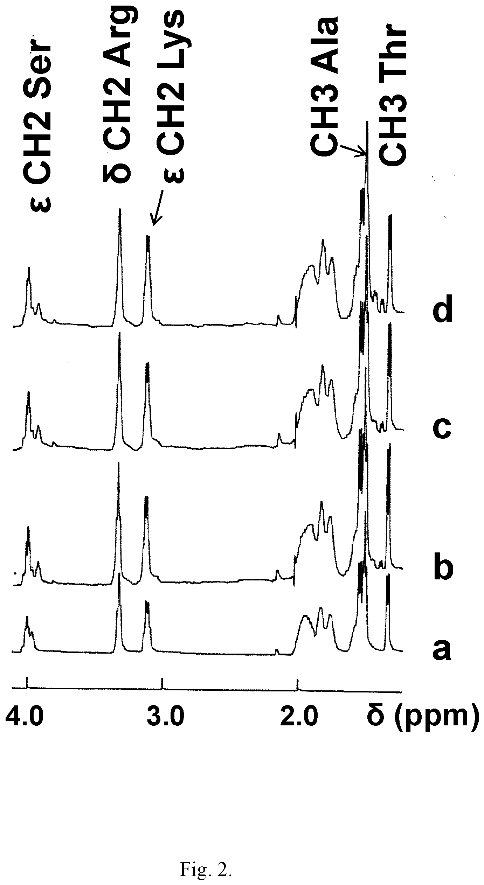

¹H-NMR spectroscopy was employed to analyze relative degradation of PIK7 (the claimed peptide) and MLC₁₁₋₁₉ (the structural analogue) in human plasma. ¹H-NMR spectroscopy allows simultaneous direct monitoring the amounts of original full-length peptide and its fragments during the course of enzymatic hydrolysis (Isakova et al., 1986). Stability of a peptide, its degradation pathway(s) and a half-life estimate are available from a comparison of spectra obtained at different time points after the addition of a peptide to plasma. ¹H-NMR spectra were obtained using a WM-500 spectrometer (Bruker, Germany) at 37°C. Chemical shifts in ¹H-NMR spectra were measured relative to an internal standard (2,2-dimethyl-2-silapentane-5-sulfonate sodium salt). The signals were referenced by the double-resonance method. The resonances from peptide protons were identified by the differential spectroscopy method, i.e. the spectrum of blood plasma was subtracted from each spectrum obtained in the presence of peptide of interest. Peptide concentrations were 3 mg/ml. Although the half-life values obtained in in-vitro experiments would differ from those in the bloodstream (because much higher peptide concentrations are used in in-vitro experiments and plasma enzymatic activity in vitro would likely be inferior to that under normal physiological conditions), these in-vitro degradation data provide insights regarding peptide relative stability to proteolytic degradation and show that the half-life of the claimed peptide is significantly higher as compared to the most close structural analogue, MLC₁₁₋₁₉. Thus, MLC₁₁₋₁₉ half-life is 32 min (Fig. 1) whereas the claimed peptide half-life is at least 7 hours (Fig. 2).

Fig. 1 shows that in 25-min incubation of MLC₁₁₋₁₉ in human plasma in vitro, apart from the signals attributed to the N-terminal Lys¹¹ protons, there are clearly detected signals (indicated by arrows) attributed to C^{α} H atoms of liberated Lys. These indicate the peptide N-terminus degradation. However, even after 320 min of incubation in human plasma, the initial spectrum of the claimed peptide (Fig. 2) does not show significant changes indicating high stability of the peptide in human plasma with a half-life > 320 min. Therefore, the claimed peptide has an increased life-span in plasma that allows for long-lasting maintenance of its effective concentrations necessary to decrease the endothelial hyperpermeability under emergency conditions. Moreover, an increased life-time allows attaining the desired therapeutic effect with lower (as compared to homologues) peptide concentrations.

### Example 3. Endothelial monolayer permeability in vitro

Cells were grown in 24-well plates (Greiner Bio-one, Austria) equipped with special inserts to form inner (upper) chambers with a porous bottom (the pore diameter of 0.4 µm, pore density of 10⁸/cm²) in DMEM until monolayer was formed (3-4 days). Successful monolayer was checked by measuring FITC-dextran diffusion for 1 hour through inserts with cell monolayers vs inserts without cells ('blank' inserts). Experiments were performed in triplicates when tight monolayers were formed. On the day of experiment, the cells were deprived for 1 h in DMEM supplemented with MLCK inhibitors or without inhibitors (control). After deprivation, FITC-dextran was added to the upper compartment to a final concentration of 0.05 mg/ml. In 30 and 60 min, a 10-µl aliquot was withdrawn from the lower compartment and mixed with 100 µl phosphate buffered saline pH 7.3 in a 96-well plate to estimate the endothelial monolayer baseline permeability. A hyperpermeability response was induced by 100 nM thrombin. During the next 3 hrs, every 30 min, aliquots were taken from the lower compartment, and intensity of FITC fluorescence was measured using a Victor 3X plate reader (Perkin Elmer, USA) to plot the dynamics of the FITC fluorescence changes.

As shown, 100 nM thrombin induces the endothelial hyperpermeability response as reflected by an increase in 70 kDa FITC-dextran diffusion through the endothelial monolayer and an incremental increase in the fluorescence in the lower compartment of the diffusion chamber. Pre-incubation of endothelial cells with MLCK peptide inhibitors (100 µM) prevented the thrombin-mediated endothelial hyperpermeability. Under these experimental conditions, all tested inhibitors effectively abolished FITC-dextran hyperpermeability. Therefore, the performance of the claimed peptide, PIK7, is not inferior to performances of both the analogue, MLC₁₁₋₁₉, and the prototype, PIK2, peptides.

### Example 4. The claimed peptide (PIK7), the prototype (PIK2), and the analogue (MLC₁₁₋₁₉) inhibitory activity assessment

Antagonistic effects of MLCK peptide inhibitors on the thrombin-mediated RLC phosphorylation in EA.hy926 endothelial cells were tested as follows. The cells were grown in 35-mm dishes in DMEM at 37°C and the 5% CO₂ atmosphere until the monolayer formed. Then, the cells were serum-starved while simultaneously incubated with 100 µM MLCK inhibitors at 37°C, 5% CO₂ for 1 hr (control, 0 min). Next, the cells were stimulated with 100 nM thrombin for 20 min and collected on ice, i.e. the medium was decanted, the cells were washed with cold PBS and lysed with 100 µL of Laemmli denaturing electrophoresis buffer. The sample proteins were separated by SDS-PAGE and proceeded to immunoblotting with anti-total, anti-monophosphorylated, and anti-diphosphorylated RLC. Membranes were developed using an ECL kit (Thermo Scientific, USA) and ECL signal was acquired and digitized using a Fusion SL4 instrument (Vilber Lourmat, France). Images were processed with the ImageJ software, which allows digitizing immunoblot band intensities in arbitrary units. Levels of RLC mono- and diphosphorylation were normalized by the total RLC signal. Data for mono- and diphosphorylation in each sample were pooled and represented as net myosin RLC phosphorylation in endothelial cells.

Fig. 4 shows that the claimed peptide (PIK7) inhibits RLC phosphorylation in endothelial cells and its efficacy is superior to both the prototype, PIK2, and the analogue, MLC₁₁₋₁₉.

### Example 5. Acute toxicity and tolerance of PIK2 and PIK7 in experimental animals

The study was conducted in 82 BALB/c mice (both males and females, body weight 18-24 g) obtained from the animal facility of the Scientific Center for Biomedical Technologies of the Federal Medico-Biological Agency of the Russian Federation (SCBMT). The animals were certified and otherwise healthy. The animals were kept in T-3 cages (5-10 animals per cage) with artificial 12 hrs light-dark cycles and 16x per hour forced air ventilation, temperature of 20-22°C and 50-65% humidity on wood chips sterilized by hot air. The animals had free access to drinking water and PK-120-1 mouse cubes (GOST P50258-92; Laboratorsnab LLC, Russia).

PIK2 and PIK7 peptides were dissolved in water and administered to mice as a single intraperitoneal injection. The animals were followed for 14 days after peptide injections. An increasing dosage scheme was applied where each tested dose surpassed the previous dose by 50%. The lowest dose of the active substance was determined that caused animal death. For PIK2, this dose was 27 mg/kg; for PIK7, the dose was 1274 mg/kg, i.e. 47-fold higher than that with PIK2. The experiments showed that a single intraperitoneal injection of a toxic dose of either PIK2 or PIK7 was associated with movement retardation and death within the following 24 hrs.

Tolerance of a single intravenous injection of either PIK2 or PIK7 was tested in Chinchilla rabbits. PIK2 was tested in a group of 4 male Chinchilla rabbits (body weight 3.43-3.82 kg). PIK7 was tested in a group of 4 male Chinchilla rabbits (body weight 2.69-2.89 kg). The tested dose of PIK2, 5 mg/kg, exceeded 5-fold the highest proposed therapeutic dose for humans (1 mg/kg). PIK2 dose of 5 mg/kg was calculated individually for each rabbit and administered into marginal ear vein in a final volume of 1.7-1.9 mL in sterile physiological saline. Stock concentration of the tested substance was 10 mg/mL. The tested dose of PIK7 (10 mg/kg) was 10-fold higher than the highest proposed therapeutic dose for humans. PIK7 stock solution (11.1 mg/mL) was prepared and administered to rabbits in a final volume of 2.4-2.6 mL.

The animals were kept at a standard animal quarter setting and had free access to drinking water and K-122 cubed feed. During the 24-hrs testing period, the overall health status and behavior were monitored (appetite, motion activity, hair coat condition).

Before injection of test substances (baseline) and 4 hrs and 24 hrs after the injection, the following parameters were tested in whole venous blood samples (3.0-3.5 mL) collected from a marginal ear vein - red blood cell (RBC) count, hematocrit, hemoglobin level, mean RBC volume, mean corpuscular hemoglobin, total leukocyte, lymphocyte, and granulocyte counts and their percentages, platelet count, and mean platelet volume. In parallel, the following biochemical parameters were measured in serum - total protein, bilirubin, urea, creatinine, glucose, total cholesterol, triglycerides, and activities of alkaline phosphatase, lactate dehydrogenase, aspartate aminotransferase, and alanine aminotransferase. Blood cell count was performed using an Abacus junior vet (Austria) hematological cell counter; biochemical studies were performed using a Targa-2000 (Italy) instrument.

The study showed that on the second minute after intravenous injection of PIK2 at a dose of 5 mg/kg, rabbits demonstrated irregular breathing, shortness of breath, muscular stiffness, prone position with straighten extremities. In 30 min after the injection, two of four rabbits recovered demonstrating no signs of altered health status or unconventional behavior. The other two rabbits died on the 7^{th} and 10^{th} minute, respectively, after the injection.

After intravenous injection of PIK7 to rabbits at a dose of 10 mg/kg, all animals survived and there were no significant changes either in the overall health status or behavior; the physical activity and appetite were not disturbed and the hair coat was unaltered. In 24 hrs after administration of PIK7 to rabbits, there was slight but statistically significant decrease in the hemoglobin level (131±5 g/L vs 120±7 g/L), hematocrit (38.81±1.49 % vs 35.90±0.18 %) and total plasma protein (73.6±2.58 g/L vs 69.2±0.47 g/L) accompanied by an increase in triglyceride levels (1.63 mmol/L vs 2.79 mmol/L). Other standard hematological and biochemical parameters were not altered after PIK7 administration. Therefore, PIK7 tolerance by rabbits is significantly superior to that of PIK2. PIK7 is not lethal to rabbits at dose that is 10-fold higher than the proposed therapeutic dose in humans (1 mg/kg).

### Literature cited.

Abramov A. A., Az'muko A. A., Bespalova Zh.D., Bushuev V.N., Vilitkevich E.L., Kazakova O.A., Kapelko V.I., Lakomkin V.L., Molokoedov A.S., Samsonov M.V., Sidorova M.V., Khapchaev A.Y., Shirinsky V.P. Amide of nonapeptide that prevents hyperpermeability of vascular endothelium. Russian Federation patent RU 2493164 C1, issued 20.09.2013.
Bespalova Zh.D., Bushuev V.N., Kulikova T.G., Marchenko A.V., Molokoedov A.S., Sekridova A.V., Sidorova M.V., Stepanova O.V., Shirinsky V.P. 2009. Amide of nonapeptide capable of preventing increased permeability of vascular endothelium. Russian Federation patent RU 2402565 C1, issued 27.10.2010. Chilcoat, C.D., Y. Sharief, and S.L. Jones. 2008. Tonic protein kinase A activity maintains inactive beta2 integrins in unstimulated neutrophils by reducing myosin light-chain phosphorylation: role of myosin light-chain kinase and Rho kinase. J Leukoc Biol. 83 :964-971.
Clayburgh, D.R., T.A. Barrett, Y. Tang, J.B. Meddings, L.J. Van Eldik, D.M. Watterson, L.L. Clarke, R.J. Mrsny, and J.R. Turner. 2005. Epithelial myosin light chain kinase-dependent barrier dysfunction mediates T cell activation-induced diarrhea in vivo. The Journal of clinical investigation. 115:2702-2715.
Glyn, M.C., and B.J. Ward. 2000. Contraction in cardiac endothelial cells contributes to changes in capillary dimensions following ischaemia and reperfusion. Cardiovasc Res. 48:346-356.
Hruby, V.J., and X. Qian. 1994. Approaches to the asymmetric synthesis of unusual amino acids. Methods Mol Biol. 35:249-286.
Hunt, J.T., D.M. Floyd, V.G. Lee, D.K. Little, and S. Moreland. 1989. Minimum requirements for inhibition of smooth-muscle myosin light-chain kinase by synthetic peptides. Biochem J. 257:73-78.
Isakova, O.L., N.F. Sepetov, Zh.D. Bespalova, V.N. Bushuev, V.A. Vinogradov. 1986. Study of peptide degradation in the serum by 1H-NMR. Bioorg Khim. 12:106-11.
Khapchaev, A.Y. M.V. Samsonov, O.A. Kazakova, E.L. Vilitkevich, M.V. Sidorova, A.A. Az'muko, A.S. Molokoedov, Zh.D. Bespalova, and V.P. Shirinsky. 2012. Suppression of Vascular Endothelium Hyperpermeability by Cell-Permeating Peptide Inhibitors of Myosin Light Chain Kinase. Biophysics 57:587-591.
Khapchaev, A.Y., O.A. Kazakova, M.V. Samsonov, M.V. Sidorova, V.N. Bushuev, E.L. Vilitkevich, A.A. Az'muko, A.S. Molokoedov, Z.D. Bespalova, and V.P. Shirinsky. 2016. Design of peptidase-resistant peptide inhibitors of myosin light chain kinase. J Pept Sci. 22:673-681.
Khapchaev, A.Y., and V.P. Shirinsky. 2016. Myosin Light Chain Kinase MYLKl: Anatomy, Interactions, Functions, and Regulation. Biochemistry (Mosc). 81:1676-1697.
Lukas, T.J., S. Mirzoeva, U. Slomczynska, and D.M. Watterson. 1999. Identification of novel classes of protein kinase inhibitors using combinatorial peptide chemistry based on functional genomics knowledge. Journal of medicinal chemistry. 42:910-919.
Marchenko, A.V., E.O. Stepanova, A.V. Sekridova, M.V. Sidorova, V.N. Bushuev, Zh.D. Bespalova, V.P. Shirinsky. 2010. Novel peptide inhibitors of myosin light chain kinase suppress the hyperpermeability of vascular endothelium. Biophysics. 55:926-930.
Owens, S.E., W.V. Graham, D. Siccardi, J.R. Turner, and R.J. Mrsny. 2005. A strategy to identify stable membrane-permeant peptide inhibitors of myosin light chain kinase. Pharm Res. 22:703-709.
Pearson, R.B., L.Y. Misconi, and B.E. Kemp. 1986. Smooth muscle myosin kinase requires residues on the COOH-terminal side of the phosphorylation site. Peptide inhibitors. J Biol Chem. 261:25-27.
Sekridova, A.V., M.V. Sidorova, A.A. Az'muko, A.S. Molokoedov, V.N. Bushuev, A.V. Marchenko, O.V. Shcherbakova, V.P. Shirinsky, Zh.D. Bespalova. 2010. Peptidase-resistant peptide inhibitors of myosin light chain kinase. Russian Journal of Bioorganic Chemistry. 36:461-467.
Shirinskii V.P. 2011. [Molecular physiology of the endothelium and mechanisms of vascular permeability]. Usp Fiziol Nauk. 42:18-32.
Wainwright, M.S., J. Rossi, J. Schavocky, S. Crawford, D. Steinhorn, A.Y. Velentza, M. Zasadzki, V. Shirinsky, Y. Jia, J. Haiech, L.J. Van Eldik, and D.M. Watterson. 2003. Protein kinase involved in lung injury susceptibility: evidence from enzyme isoform genetic knockout and in vivo inhibitor treatment. Proceedings of the National Academy of Sciences of the United States of America 100:6233-6238.

## Claims

1. Proteolytically stable nonapeptide of formula
H-Lys-*D*-Lys-Arg-Ala-Ala-Arg-Ala-Thr-Ser-NH₂ (I)
capable of preventing vascular endothelial hyperpermeability.

## Patentansprüche

1. Proteolytisch stabiles Nonapeptid der Formel H-Lys-*D*-Lys-Arg-Ala-Ala-Arg-Ala-Thr-Ser-NH₂ (I) das in der Lage ist, die vaskuläre endotheliale Hyperpermeabilität zu verhindern.

## Revendications

1. Nonapeptide stable sur le plan protéolytique de formule
H-Lys-D-Lys-Arg-Ala-Ala-Arg-Ala-Thr-Ser-NH₂ (I)
capable de prévenir l'hyperperméabilité endothéliale vasculaire.
